(19) Europäisches Patentamt
European Patent Office
Office européen des brevets

(11) Numéro de publication: **0 217 778 B1**

(12) **FASCICULE DE BREVET EUROPEEN**

(45) Date de publication de fascicule du brevet: **20.05.92**

(51) Int. Cl.5: **A61K 31/275**, A61K 9/52

(21) Numéro de dépôt: **86870136.8**

(22) Date de dépôt: **25.09.86**

# (54) Formes galéniques à libération prolongée du vérapamil, leur fabrication et médicaments les contenant.

(30) Priorité: **30.09.85 LU 86099**

(43) Date de publication de la demande:
**08.04.87 Bulletin 87/15**

(45) Mention de la délivrance du brevet:
**20.05.92 Bulletin 92/21**

(84) Etats contractants désignés:
**AT BE CH DE FR GB IT LI LU NL SE**

(56) Documents cités:
**EP-A- 0 063 266**
**EP-A- 0 142 877**
**EP-A- 0 153 104**

**CHEMICAL ABSTRACTS, vol. 97, no. 8, 23 août 1982, page 323, résumé no. 60921p, Columbus, Ohio, US; P. HEINZ: "Water vapor and oxygen permeability of various tablet coatings", & Acta Pharm. Technol. 1982, 28(1), 21-33**

(73) Titulaire: **"PHARLYSE", Société Anonyme**
**Boulevard Royal, 2**
**Luxembourg(LU)**

(72) Inventeur: **de Boeck, Arthur**
**Steenweg op Edingen, 61**
**B-1540 Herne(BE)**
Inventeur: **Baudier, Philippe**
**Avenue Blücher, 10**
**B-1410 Waterloo(BE)**
Inventeur: **Fossion, Jacques**
**Rue du Cours d'Eau, 18**
**B-1428 Braine-l'Alleud(BE)**

(74) Mandataire: **Fobe, Edouard et al**
**Bureau Vander Haeghen S.A. Rue Colonel Bourg 108 A**
**B-1040 Bruxelles(BE)**

Il est rappelé que: Dans un délai de neuf mois à compter de la date de publication de la mention de la délivrance du brevet européen, toute personne peut faire opposition au brevet européen délivré, auprès de l'Office européen des brevets. L'opposition doit être formée par écrit et motivée. Elle n'est réputée formée qu'après paiement de la taxe d'opposition (art. 99(1) Convention sur le brevet européen).

EP 0 217 778 B1

## Description

La présente invention est relative à de nouvelles formes galéniques à libération programmée et prolongée du vérapamil. Elle concerne de telles formes galéniques particulièrement stables dans des conditions de températures élevées telles qu'elles peuvent se rencontrer, de façon accidentelle, dans les pays à climat tempéré et, de façon beaucoup plus prévisible et fréquente, dans des pays à climat tropical. La stabilité de ces nouvelles formes réside dans leur capacité à conserver, inchangées, à la fois la cinétique de libération du vérapamil ainsi que la quantité totale de ce produit libérée, après un séjour dans de telles conditions de température.

La présente invention concerne également la fabrication de ces nouvelles formes galéniques et les médicaments qui les renferment.

Le vérapamil ou 5-(3,4-diméthoxyphényléthyl)-méthylamino-2-(3,4-diméthoxyphényl)-2-isopropyl valéronitrile de formule I est connu depuis plus de 20 ans et sa synthèse est décrite dans le brevet belge n° 615.816 correspondant au brevet U.S. Dengel n° 3.261.859.

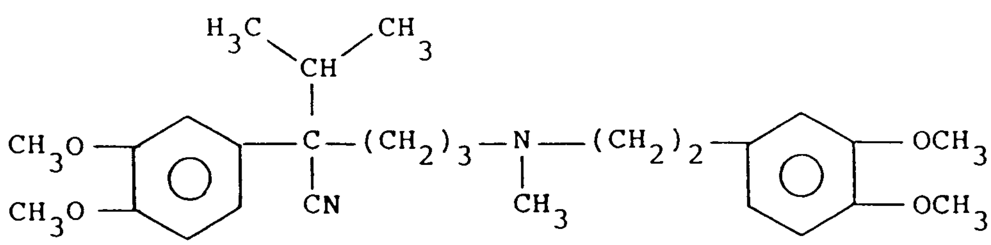

(I)

Le chlorhydrate de vérapamil est utilisé en médecine pour ses remarquables propriétés d'antagoniste de la pénétration intracellulaire du calcium. Ceci en fait un médicament intéressant dans l'angine de poitrine lorsque la crise est liée à un spasme coronarien et lorsque des effets indésirables des produits beta-adrénolytiques, tels que le propranolol, le timolol, l'aténolol et le pindolol sont à craindre. Il est également utile dans le traitement de l'hypertension et des troubles du rythme cardiaque.

Il est connu de l'homme de l'art que l'action pharmacologique du vérapamil est proportionnelle à sa concentration plasmatique (Br J. Clin. Pharmac (1981), 12, 397-400) et que la zone thérapeutique optimale est comprise entre 100 ng/ml et 400 ng/ml de plasma.

L'inconvénient majeur rencontré lors d'un traitement à base de vérapamil est dû à son temps de demi-vie plasmatique très court (2 à 4 heures), ce qui nécessite plusieurs administrations quotidiennes, espacées de 6 heures seulement. Des temps d'administration aussi rapprochés rendent le traitement fort astreignant, voire impossible à suivre, surtout la nuit. De plus, on constate, après chaque administration d'une forme galénique de vérapamil à libération immédiate, c'est-à-dire en général quatre fois par jour, une succession de croissances et de décroissances rapides des taux plasmatiques. L'organisme et notamment l'organecible, en l'occurrence le coeur, sont ainsi soumis alternativement des surdosages et à des sous-dosages du médicament.

Afin de pallier ces inconvénients, une première forme galénique à libération prolongée du vérapamil dénommée "Isoptine retard®" se présentant sous la forme d'un comprimé constitué d'une matrice hydrophile a été mise sur le marché. Bien que cette forme galénique à libération prolongée permette d'éliminer les pics de concentrations, la biodisponibilité du vérapamil au départ de cette forme galénique est extrêmement faible, comme on le verra plus loin, de telle sorte qu'elle ne permet pratiquement pas d'atteindre des taux plasmatiques situés dans la zone thérapeutique optimale et rend par conséquent difficile l'obtention de l'effet clinique souhaité.

Un autre type de formes galéniques, constituées de pellets ou microgranules enrobés et produisant également une libération prolongée du vérapamil, a été développé ultérieurement (demande de brevet européen n° 86870129.3 déposée par la demanderesse le 17 septembre 1986 et jubliée sous le n° 0 216 743). Bien que ce nouveau type de formes galéniques présente une biodisponibilité remarquable alliée à une excellente cinétique de libération prolongée du vérapamil, il révèle, par contre, un manque de stabilité de cette cinétique dans des conditions de conservation rigoureuses par leurs températures élevées, mais absolument indispensables à la sécurité d'utilisation de ce type de préparation. En effet, au cours d'essais de stabilité auxquels ces formes galéniques selon la demande de brevet européen précitée ont été soumises, c'est-à-dire à des températures comprises entre 45 et 70° Celsius, la cinétique de libération du

vérapamil s'est fortement altérée (voir les figures 1, 2 et 3 ci-annexées). Cette altération s'est manifestée, d'une part, par une diminution très importante de la vitesse de libération du vérapamil et, d'autre part, par une libération incomplète de ce vérapamil, la fraction libérée ne dépassant pas, dans certains cas, 50 % de la dose libérable.

Les conséquences de cette libération altérée sont :

1. que le produit ne répondra plus aux normes de libération fixées "in vitro", ce qui implique, à l'occasion d'un recontrôle, le retrait du marché et/ou la destruction par le fabricant des lots du médicament ayant subi ce phénomène,

2. que le produit ainsi altéré ne sera plus capable de produire l'effet thérapeutique escompté, ceci étant d'autant plus dangereux, d'une part, qu'il s'agit d'un médicament utilisé dans le domaine cardio-vasculaire et, d'autre part, que l'aspect extérieur du médicament altéré ne permet pas de déceler cette altération.

Enfin on connaît par le document EP-A-153104 des formes administrables par voie buccale dans lesquelles des unités individuelles contenant une substance active telle que du vérapamil sont revêtues d'un premier film et éventuellement d'un deuxième film extérieur. Les unités individuelles peuvent être des granules contenant à titre de liant de la polyvinylpyrrolidone, de la gélatine ou de la polyvinylcellulose. Dans cet article il n'est jamais fait allusion à la notion d'agent mouillant.

Le premier film comprend une combinaison homogène d'un agent filmogène dispersible dans l'eau et d'une substance polymérique qui améliore la compression du revêtement.

L'agent filmogène peut, par exemple, être l'hydroxypropylméthylcellulose phtalate, des polymères acryliques, des copolymères acryliques ou des mélanges de ceux-ci, tandis que la substance polymérique est de préférence un polymère soluble dans l'eau tel que l'hydroxypropylcellulose.

Dans cette demande, seuls des exemples de films comprenant un seul polymère, à savoir un polymère acrylique insoluble dans l'organisme et un adjuvant, sont donnés.

Ce document n'enseigne pas un revêtement permettant une stabilité remarquable de la cinétique de libération prolongée de vérapamil puisqu'il n'y est jamais fait référence à une membrane microporeuse constituée :

- d'au moins un adjuvant pharmacologiquement acceptable, et
- d'au moins un mélange filmogène composé, d'une part, d'au moins un polymère du type acrylique et/ou méthacrylique insoluble dans l'organisme et, d'autre part, d'au moins une substance polymérique ou non mais de nature non acrylique ou méthacrylique.

La présente invention a pour objet une nouvelle forme galénique à libération prolongée du vérapamil, cette forme galénique possédant une excellente cinétique de libération du vérapamil et une haute biodisponibilité de ce vérapamil, lesquelles caractéristiques essentielles de la nouvelle forme galénique s'avèrent absolument stables au cours de sa conservation non seulement à température ambiante, mais également à des températures élevées, telles que celles comprises entre 45 et 70° Celsius.

Conformément à la présente invention, les nouvelles formes galéniques à libération prolongée du vérapamil, qui sont constituées de microgranules contenant un sel pharmacologiquement acceptable d'addition du vérapamil avec un acide, tel que le chlorhydrate de vérapamil, comme substance active, associé à au moins un mouillant, lesdites microgranules étant enrobées d'une membrane, sont essentiellement caractérisées en ce que

les microgranules contiennent, outre le sel de vérapamil, au moins un agent mouillant choisi parmi les sucres, la polyvinylpyrrolidone, les esters d'acides gras en $C_{12}$ à $C_{20}$ du saccharose ou du xylose, les glycérides du saccharose, les esters d'acides gras de polyoxyéthylène, les éthers d'alcools gras et de polyoxyéthylène, les esters de sorbitane, les esters de sorbitane polyoxyéthylénés, les glycérides-polyglycides, les esters d'alcools-polyglycides et les lécithines, et en ce que

les microgranules sont enrobées d'une membrane microporeuse constituée d'au moins un adjuvant pharmacologiquement acceptable choisi parmi les plastifiants, pigments, charges, agents mouillants, agents lubrifiants, agents antistatiques et agents anti-mousse, et d'au moins un mélange filmogène composé, d'une part, d'au moins un polymère de type acrylique et/ou méthacrylique insoluble dans l'organisme et, d'autre part, d'une substance, polymérique ou non, mais de nature non acrylique ou méthacrylique, insoluble en milieu gastrique acide mais entérosoluble, ladite substance étant choisie parmi l'acétophtalate de cellulose, l'acétophtalate de polyvinyle, les phtalates d'hydroxypropylméthylcellulose, les poly (éther méthylvinylique / anhydride maléïque), les acides choliques et les acides gras en $C_{10}$ à $C_{20}$ ; de sorte que la forme galénique est stable à la chaleur.

Cette membrane microporeuse permet une libération prolongée du vérapamil suivant une cinétique dont les caractéristiques ne se modifient pas après chauffage jusqu'à 70° Celsius.

En plus du ou des mouillants, les microgranules peuvent également contenir au moins un excipient qui peut être choisi parmi les suivants :

- les celluloses microcristallines, telles que les produits Avicel® (FMC, U.S.A.) ;
- les méthylcelluloses, éthylcelluloses (Ethocel® ou Aqua-Coat®), carboxyméthylcelluloses, hydroxyéthylcelluloses (Natrosol®, Hercules, U.S.A.), hydroxypropylcelluloses (Klucels®, Hercules, U.S.A.) ;
- les amidons.

Selon une autre particularité de l'invention, la membrane microporeuse dont sont enrobés les microgranules contenant le sel de vérapamil est constituée d'un mélange filmogène composé, d'une part, d'au moins un polymère du type acrylique et/ou méthacrylique insoluble dans l'organisme et, d'autre part, d'au moins une substance polymérique ou non, insoluble en milieu gastrique acide mais entérosoluble et de nature non acrylique ou méthacrylique, ce mélange étant associé à au moins un adjuvant pharmacologiquement acceptable choisi parmi les plastifiants, pigments, charges, agents mouillants, lubrifiants, antistatiques et agents anti-mousse.

Les microgranules contenant la substance active se présentent sous forme de sphérules, dont le diamètre est compris entre 0,05 mm et 3 mm, de préférence entre 0,1 mm et 2 mm.

Parmi les mouillants associés au chlorhydrate de vérapamil dans les microgranules, on peut citer, en particulier, les suivants :

- le saccharose, le mannitol, le sorbitol;
- les lécithines :
- les polyvinylpyrrolidones ;
- les esters d'acide gras en $C_{12}$ à $C_{20}$ du saccharose ou sucroesters (Gattefossé, France) et crodestas® (Croda, Grande Bretagne) ;
- les glycérides en $C_{12}$ à $C_{20}$ du saccharose ou sucroglycérides ;
- les esters de xylose ou xylites ;
- les glycérides polyoxyéthyléniques ;
- les esters d'acides gras et de polyoxyéthylène (Tefose® de Gattefossé, France: Crémophores® de BASF, RFA) ;
- les éthers d'alcool gras et de polyoxyéthylène (Brijs, Renex® de Atlas, U.S.A.; Eumulgines® de Henkel) ;
- les esters d'acides gras et du sorbitane (Spans® de Atlas, U.S.A.) ;
- les esters polyoxyéthylénés d'acides gras et du sorbitane (Tweens® de Atlas, U.S.A.) ;
- les glycérides-polyglycides et esters d'alcoolspolyglycides (Gelucires® de Gattefossé, France).

Parmi les polymères insolubles du type acrylique et/ou méthacrylique contenus dans le mélange filmogène, on peut citer, en particulier, les polyacrylates et polyméthacrylates du type Eudragit®, tels que les Eudragit E30D®, Eudragit RS® et Eudragit RL® de la firme Röhm Pharma (RFA) (Eudragit® sont des copolymères ester éthylique d'acide acrylique/ester methylique d'acide méthacrylique - voir EP-A-153 104).

Parmi les substances, polymériques ou non, insolubles en milieu gastrique acide, mais entérosolubles, on peut citer, en particulier, l'acétophtalate de cellulose, l'acétophtalate de polyvinyle tel que le PVAP® de la firme Colorcon (U.S.A.), les phtalates d'hydroxypropylméthylcellulose, tels que les HP 50®, HP 55® et HP 55 F® de la firme Shin-Etsu (Japon), les poly (éther méthylvinylique/anhydride maléique) tels que les Gantrez® de la firme GAF (U.S.A.), les acides choliques et les acides gras en $C_{10}$ à $C_{20}$.

Le ou les polymères peuvent être associés dans la membrane microporeuse à au moins un adjuvant choisi parmi les suivants :

- plastifiants, tels que la triacétine, le dibutylphtalate, le dibutylsébaçate, les esters d'acide citrique, les polyéthylèneglycols, les polypropylèneglycols et la polyvinylpyrrolidone ;
- pigments éventuellement colorés, tels que les oxydes de fer et l'oxyde de titane ;
- charges, telles que le lactose et le saccharose ;
- mouillants, tels que les agents tensio-actifs des types Span® et Tween®, à savoir des esters partiels d'acides gras (acides laurique, palmitique, stéarique et oléique) et d'anhydrides d'hexitols dérivés de sorbitol contenant éventuellement des chaînes polyoxyéthyléniques, de préférence les agents tensio-actifs du type Tween®, notamment le Tween 80®, ainsi que les polyéthylèneglycols ;
- lubrifiants, tels que le stéarate de magnésium et le talc;
- agents anti-mousse, tels que les huiles de silicone ;
- agents antistatiques, tels que l'oxyde d'aluminium colloïdal.

Outre le ou les polymères et la substance insoluble dans le milieu gastrique acide, mais entérosoluble, la membrane microporeuse contient, de préférence, du talc et/ou du stéarate de magnésium à titre de lubrifiant, de la polyvinylpyrrolidone à titre de plastifiant, du bioxyde de titane à titre de pigment, du Tween 80® comme mouillant, de l'oxyde d'aluminium colloïdal comme agent antistatique et de l'huile de silicone comme agent anti-mousse.

Le poids de la membrane microporeuse peut être de 2 à 35 %, de préférence de 5 à 22 % de celui des microgranules. Ces derniers peuvent contenir du chlorhydrate de vérapamil à raison de 10 à 95 % en poids, de préférence de 30 à 85 % en poids.

La membrane microporeuse peut contenir de 25 à 95 % et, de préférence, de 30 à 90 % de polymère ou mélange de polymères.

L'invention concerne également un médicament contenant du vérapamil à titre d'ingrédient à libération prolongée, ce médicament étant constitué de microgranules contenant du vérapamil, de préférence sous forme de chlorhydrate, enrobés d'une membrane microporeuse, ces microgranules enrobés étant contenus dans des gélules, sachets ou distributeurs de doses, ou agglomérés sous forme de comprimés.

La présente invention concerne également un procédé d'obtention des nouvelles formes du vérapamil à libération retardée et/ou prolongée dans le tractus gastro-intestinal, ce procédé consistant à préparer au préalable des microgranules et à les enrober d'une membrane microporeuse.

Diverses techniques connues sont utilisables pour obtenir les microgranules contenant du chlorhydrate de vérapamil, comme décrit dans la demande de brevet européen n° 86870129.3 du 17 septembre 1986 de la demanderesse. Ils peuvent être obtenus soit par sphéronisation d'un extrudat, soit par saupoudrage et/ou pistolage dans une turbine ou dans un granulateur du type "CF granulator", soit encore par mélange dans un granulateur planétaire.

La membrane microporeuse peut être appliquée sur les microgranules secs et calibrés au diamètre voulu par passage au travers des tamis appropriés, par pulvérisation du mélange filmogène et d'au moins un des adjuvants susdits. Cette pulvérisation peut s'effectuer par pistolage ou pulvérisation de la dispersion susdite dans une turbine à dragéifier ou dans un appareil à lit fluidisé.

Selon une forme de réalisation exemplative de l'invention, la membrane microporeuse est obtenue au départ d'une dispersion aqueuse contenant, en poids :

| | |
|---|---|
| 10 à 70 % | d'Eudragit E30D® (polymère) ; |
| 5 à 70 % | de phtalate d'hydroxypropylméthylcellulose HP 55 F® (substance insoluble en milieu gastrique acide et entérosoluble) ; |
| 1 à 15 % | de talc (lubrifiant) ; |
| 1 à 15 % | de polyvinylpyrrolidone (plastifiant) ; |
| 0,05 à 2 % | d'oxyde d'aluminium colloïdal (antistatique) ; |
| 0,01 à 2 % | de Tween 80® (mouillant) ; |
| 0,01 à 2 % | d'huile de silicone (agent anti-mousse) ; |
| 10 à 70 % | d'eau (véhicule). |

L'ensemble des caractéristiques et avantages de l'invention seront mieux compris par l'homme de l'art en se référant à la description qui va suivre de modes de réalisation particuliers donnés à titre d'exemples non limitatifs de la nouvelle forme galénique du vérapamil, de son procédé de fabrication et de ses applications thérapeutiques, en particulier en relation avec des contrôles pharmacocinétiques utilisant cette nouvelle forme galénique. Les études de la stabilité de la cinétique de libération de ces nouvelles formes galéniques au cours de leur conservation à diverses températures permettent également de comprendre l'intérêt et les avantages de la présente invention.

## Exemples de préparation de la nouvelle forme galénique

### EXEMPLE 1

1.a. Fabrication des microgranules

On a utilisé les ingrédients suivants :

| | |
|---|---|
| Vérapamil HCl | 395 g |
| Sucroester WE 15® (Gattefossé) | 50 g |
| Avicel PH 101 *® | 50 g |
| Polyvinylpyrrolidone K30® | 5 g |
| | 500 g |

* cellulose microcristalline de la firme FMC, U.S.A.

Après avoir introduit les produits précités en poudre dans un mélangeur planétaire et les avoir granulés par addition de 75 g d'eau distillée, la masse plastique obtenue a été extrudée à travers des trous d'un diamètre de 1 mm de la filière cylindrique d'une extrudeuse (Alexanderwerk). Les petits cylindres obtenus ont été ensuite rendus sphériques par sphéronisation dans un appareil de type Marumerizer. Après séchage pendant 24 h dans une étuve ventilée portée a 50° C, la fraction de microgranules dont le diamètre est compris entre 0,7 mm et 1,4 mm a été récupérée par tamisage au travers de tamis appropriés. On a ainsi obtenu 437 g de microgranules (rendement : 87 %).

1.b. Enrobage des microgranules

Sur 400 g de microgranules, dont la granulométrie est comprise entre 0,7 et 1,4 mm, on a pistolé dans un appareil a lit fluidisé de marque Aéromatic type Strea 1, 192 g de la dispersion suivante :

| | |
|---|---|
| talc (lubrifiant) | 50,00 |
| polyvinylpyrrolidone (plastifiant) | 7,50 |
| Tween 20® (mouillant) | 0,50 |
| phtalate d'hydroxypropylméthylcellulose HP 55 F® (substance entérosoluble) | 68,80 |
| Eudragit E30D® (polymère insoluble) | 410,80 |
| eau | 462,4 |
| | 1000,00 |
| Durée du pistolage : 40 minutes.<br>Séchage dans étuve à 50° C pendant 24 heures. | |

1.c. Formes pharmaceutiques

Après tamisage et contrôle du titre en chlorhydrate de vérapamil des microgranules et après avoir ajusté éventuellement ce titre à la valeur souhaitée par addition de microgranules neutres, tels que des microgranules de saccharose, la quantité calculée du mélange a été introduite dans des capsules de gélatine dure pour obtenir la dose unitaire voulue de vérapamil.

1.d. Etude de la stabilité à diverses températures - mesure de la vitesse de libération du vérapamil HCl hors des microgranules enrobés

Pour vérifier la qualité de la cinétique de libération du vérapamil, immédiatement après fabrication, on a utilisé la méthode décrite dans "The United States Pharmacopoeia (USP) XXI", édition 1985, p 1243-1244 sous le titre : Dissolution Apparatus II, le milieu de dissolution étant constitué d'un tampon pH 5,8. On a utilisé une prise d'essai de 251,5 mg de microgranules de l'exemple 1 par ballon.

Immédiatement après la fabrication, les résultats suivants ont été obtenus lors de la mesure de la cinétique de dissolution.

| Temps | % de vérapamil HCl dissous ± SD (déviation standard) |
|---|---|
| Après 1 h | 5,2 ± 0,2 |
| Après 4 h | 36,5 ± 0,7 |
| Après 8 h | 61,3 ± 1,2 |

Afin d'étudier la stabilité de la préparation, des gélules remplies de microgranules de l'exemple 1 ont été placées dans trois étuves dont la température était maintenue respectivement à 25, 50 et 70 °C. Les cinétiques de dissolution du vérapamil à partir de ces gélules ont été ensuite mesurées, suivant la même méthode de l'USP XXI déjà citée.

Après des séjours d'une durée de 7 jours, 15 jours et 1 mois dans les étuves, l'étude de la stabilité de la cinétique, basée sur les mesures des pourcentages de vérapamil libérés après 1 heure, 4 heures et 8 heures, a été effectuée (voir les figures 4, 5 et 6 ci-annexées).

Au terme de cette étude, la comparaison des figures 1, 2, 3, d'une part, et des figures 4, 5, 6 d'autre part (les microgranules de ces deux groupes ne se différenciant que par leur type de membrane microporeuse) fait apparaître clairement que la nouvelle forme, objet de la présente invention, se comporte de façon tout-à-fait différente et intéressante lors des études de stabilité à températures élevées. En effet, alors que la forme galénique précédemment décrite subit une diminution très importante de sa cinétique de libération, la forme galénique suivant la présente invention maintient, de façon surprenante, dans ces mêmes conditions, sa cinétique de libération constante. A ce jour, les raisons de cette excellente stabilité, phénomène nouveau et imprévisible, n'ont pas pu être expliquées.

1.e. Etude de la biodisponibilité

La nouvelle forme galénique selon l'invention décrite plus haut a fait l'objet d'une étude pharmacocinétique approfondie en comparaison avec une forme à libération immédiate (Isoptine®) et avec une forme à libération prolongée actuellement disponible sur le marché (Isoptine Retard®).

Cinq sujets volontaires sains ont reçu successivement et dans un ordre aléatoire chacune des trois formes, à 2 semaines d'intervalle et à des doses équivalentes (360 mg de vérapamil). La détermination de la cinétique des concentrations plasmatiques du vérapamil a été effectuée au moyen de 14 à 16 prèlèvements sanguins et a permis d'obtenir les courbes de la figure 7 ci-annexée).

Sur cette figure 7, la courbe en pointillé est relative l'Isoptine normale, la courbe en traits interrompus à la forme galénique selon l'exemple 1 et la courbe en trait plein à l'Isoptine retard.

Au terme de cette étude, les conclusions sont les suivantes :

1.e.1 Biodisponibilité

D'après les valeurs des surfaces sous les courbes concentrations plasmatiques en ordonnée - temps de 0 à 24 h en abscisse, la biodisponibilité de la forme galénique de l'exemple 1 s'avère équivalente à celle du produit à libération immédiate (puisqu'elle atteint déjà 84 % de la forme à libération immédiate alors que les valeurs n'ont pas été extrapolées au-delà de 24 h), tandis que celle du produit Isoptine Retard est nettement inférieure, puisqu'elle ne vaut que 26 % du produit à libération rapide.

1.e.2 Allure des courbes plasmatiques

Les deux formes à libération prolongée (exemple 1 et Isoptine Retard) fournissent des courbes pratiquement parallèles et dont l'allure est caractéristique de ce type de préparation galénique.

EXEMPLE 2

2.a. Fabrication des microgranules

On a utilisé les ingrédients suivants (en grammes) :

| | |
|---|---|
| vérapamil HCl | 790 |
| Avicel PH 101 *® | 50 |
| Avicel CL 611 *® | 50 |
| polyvinylpyrrolidone K30® | 10 |
| Gelucire 50/13® | 33,30 |
| Saccharose | 66,70 |
| | 1000,00 |

* celluloses microcristallines de la firme FMC, U.S.A.

Ces ingrédients ont été granulés avec 170 ml d'eau, en opérant de la même manière que dans l'exemple 1, si ce n'est que l'on a utilisé l'extrudeuse XTRUDER (Fuji-Paudal). On a obtenu 905 g de microgranules (rendement : 90,5 %).

2.b. Enrobage des microgranules

En opérant comme dans l'exemple précédent, on a pistolé, sur 700 g de microgranules de l'exemple 2, 322 g de la dispersion suivante :

| | |
|---|---|
| talc (lubrifiant) | 50,00 |
| polyvinylpyrrolidone (plastifiant) | 7,50 |
| Tween 20® | 0,50 |
| phtalate d'hydroxypropylméthylcellulose HP 55 F® | 87,50 |
| hydroxyde de soude 0,1 normale | 87,50 |
| Eudragit E30D® | 348,30 |
| eau | 418,70 |

2.c. Forme pharmaceutique

La forme pharmaceutique a été obtenue en procédant comme dans l'exemple 1.

2.d. Mesure de la cinétique de la libération du vérapamil et étude de la stabilité de la forme pharmaceutique à diverses températures

En utilisant la méthode de l'exemple 1, les résultats obtenus immédiatement après fabrication sont les suivants :

| Temps | % vérapamil HCL dissous ± SD (n = 3) |
|---|---|
| Après 1 h | 9,7 ± 0,15 |
| Après 4 h | 54,0 ± 1,07 |
| Après 8 h | 80,5 ± 1,50 |

L'étude de la stabilité à diverses températures a été menée de façon identique à celle de l'exemple 1 et les résultats sont représentés sur les figures 8, 9 et 10.

Il apparaît clairement que la forme pharmaceutique de cet exemple 2 possède également des caractéristiques de stabilité aux hautes températures tout-à-fait remarquables et également imprévisibles.

2.e. Etude de la biodisponibilité

Les résultats de cette étude sont représentés a la figure 11. Ils ont été obtenus en procédant comme dans l'exemple 1.

Les conclusions pour cet exemple 2 sont identiques à celles de l'exemple 1 et tout-à-fait remarquables puisque les valeurs trouvées sont les suivantes :

| | |
|---|---|
| forme à libération rapide (référence)<br>forme de l'exemple 2 | 100 %<br>85,9 % |
| (valeurs non extrapolées au-delà de 24 h). | |

EXEMPLE 3

3.a. Fabrication des microgranules

On a utilisé les ingrédients suivants (en grammes) :

| | |
|---|---|
| vérapamil HCl | 791 |
| Avicel PH 101 *® | 55 |
| Avicel CL 611 *® | 45 |
| polyvinylpyrrolidone K30® | 9 |
| Crodesta F160® | 100 |
| | 1000,00 |

* celluloses microcristallines de la firme FMC, U.S.A.

Ces ingrédients ont été granulés avec 145 ml d'eau, en opérant de la même manière que dans l'exemple 1, si ce n'est que l'on a utilisé l'extrudeuse XTRUDER (Fuji-Paudal). On a obtenu 913 g de microgranules (rendement : 91,3 %).

3.b. Enrobage des microgranules

En opérant comme dans les exemples précédents, on a pistolé, sur 700 g de microgranules de l'exemple 2, 322 g de la dispersion suivante :

| | |
|---|---|
| talc (lubrifiant) | 1000 |
| polyvinylpyrrolidone (plastifiant) | 150 |
| Tween 80® | 10 |
| phtalate d'hydroxypropylméthylcellulose HP 55 F® | 1662 |
| Antifoam C emulsion *® | 30 |
| Eudragit E30D® | 7258 |
| eau | 9850 |

* fabriqué par DOW CORNING (U.S.A.)

3.c. Forme pharmaceutique

La forme pharmaceutique a été obtenue en procédant comme dans l'exemple 1.

3.d. Mesure de la cinétique de la libération du vérapamil et étude de la stabilité de la forme pharmaceutique à diverses températures

En utilisant la méthode de l'exemple 1, les résultats obtenus immédiatement après fabrication sont les suivants :

| Temps | % vérapamil HCL dissous ± SD (n = 3) |
|---|---|
| Après 1 h | 9,2 ± 2,9 |
| Après 4 h | 65,8 ± 2,1 |
| Après 8 h | 93,0 ± 2,2 |

L'étude de la stabilité à diverses températures a été menée de façon identique à celle de l'exemple 1 et les résultats sont représentés sur les figures 12 et 13.

Il apparaît clairement que la forme pharmaceutique de cet exemple 3 possède également des caractéristiques de stabilité aux hautes températures tout-à-fait remarquables et également imprévisibles.

Sur le plan de la stabilité, les nouvelles formes galéniques faisant l'objet de la présente invention ont permis d'obtenir, d'une manière nouvelle et imprévisible, une préparation pharmaceutique de vérapamil à libération prolongée, dont les effets nouveaux et inattendus résident dans la constance des caractéristiques de la cinétique de dissolution du vérapamil après conservation à des températures comprises entre 25 et 70 ° C, ainsi que dans le maintien de la quantité totale de vérapamil libérée hors des microgranules et donc disponible pour l'organisme.

Ces propriétés de stabilité à la chaleur des nouvelles formes galéniques sont évidemment essentielles, car des variations de température importantes même momentanées peuvent se produire de façon imprévivisible et selon les hasards de la conservation chez le malade ou chez tout autre détenteur du médicament, et ce sans se manifester par un changement quelconque dans l'aspect du médicament.

De telles élévations importantes et momentanées ou durables de température peuvent résulter, par exemple, du dépôt du flacon de médicament près d'une source de chaleur dans l'habitation (radiateurs, soleil estival au travers d'une fenêtre, d'une véranda, etc.) ou à l'extérieur de l'habitation, par exemple dans une voiture abandonnée au soleil au cours d'un transport soit par le malade lui-même, soit par un distributeur des médicaments.

Des modifications aussi aléatoires des caractéristiques d'un médicament seraient évidemment incompatibles avec une thérapeutique sûre et efficace.

En effet, une libération trop ralentie du vérapamil aurait pour conséquence de produire des taux plasmatiques de vérapamil se situant à des niveaux inférieurs à la limite de la zone de concentrations thérapeutiques efficaces. Ce phénomène lié à une libération trop lente, est particulièrement marqué dans le cas d'une substance active dont le temps de demi-vie plasmatique court résulte d'une métabolisation rapide, telle que le vérapamil. Une vitesse de libération insuffisante du produit dans l'organisme ne parvient donc plus à contrebalancer suffisamment la vitesse de métabolisation et l'équilibre s'établit à un niveau de taux plasmatiques trop bas.

En outre, la libération incomplète du vérapamil hors des microgranules entraîne non seulement une diminution de la biodisponibilité, mais également un raccourcissement de la durée de l'effet thérapeutique, puisque la libération de la fraction disponible se fait sur un laps de temps plus court.

Sur le plan clinique, les caractéristiques de libération lente et continue du vérapamil au départ des microgranules des nouvelles formes galéniques suivant la présente invention ont permis de découvrir les avantages importants suivants :

- Meilleure adhésion du patient à son traitement du fait de la diminution du nombre de prises quotidiennes du médicament. La nouvelle forme galénique ne nécessite en général qu'une seule administration quotidienne, alors qu'un traitement équivalent au moyen de préparations libération immédiate comporte quatre prises quotidiennes, soit une prise toutes les 6 heures.
- Augmentation de l'efficacité thérapeutique : d'une part, grâce au maintien de concentrations plasmatiques efficaces durant tout l'espace de temps séparant deux prises successives de médicament, notamment pendant la nuit et, d'autre part, grâce à la suppression de la succession des pics (concentrations plasmatiques momentanément trop fortes) et de vallées (concentrations trop faibles), phénomène générateur d'effets indésirables par surdosages et de manque d'efficacité par sous-dosages.
- Risques diminués et facilité accrue dans l'adaptation de la posologie individuelle, ceci grâce à une importante diminution des fluctuations des taux plasmatiques d'un patient à l'autre.

Par conséquent, on peut affirmer que les nouvelles formes galéniques faisant l'objet de la présente invention conduisent à un nouveau médicament utile pour le traitement des troubles du rythme cardiaque, de l'hypertension et de l'angine de poitrine ou de toute autre utilisation médicale du vérapamil en administration orale.

## Revendications

1. Forme galénique du vérapamil à libération prolongée de ce composé, constituée de microgranules contenant un sel pharmacologiquement acceptable d'addition du vérapamil avec un acide, tel que le chlorhydrate de vérapamil, comme substance active, associé à au moins un agent mouillant, les microgranules étant enrobées d'une membrane, caractérisée en ce que
   les microgranules contiennent, outre le sel de vérapamil, au moins un agent mouillant choisi parmi les sucres, la polyvinylpyrrolidone, les esters d'acides gras en $C_{12}$ à $C_{20}$ du saccharose ou du xylose, les glycérides du saccharose, les esters d'acides gras de polyoxyéthylène, les éthers d'alcools gras et de polyoxyéthylène, les esters de sorbitane, les esters de sorbitane polyoxyéthylénés, les glycérides-polyglycides, les esters d'alcools-polyglycides et les lécithines, et en ce que
   les microgranules sont enrobées d'une membrane microporeuse constituée d'au moins un adjuvant pharmacologiquement acceptable choisi parmi les plastifiants, pigments, charges, agents mouillants, agents lubrifiants, agents antistatiques et agents anti-mousse, et d'au moins un mélange filmogène composé, d'une part, d'au moins un polymère de type acrylique et/ou méthacrylique insoluble dans l'organisme et, d'autre part, d'une substance, polymérique ou non, mais de nature non acrylique ou méthacrylique, insoluble en milieu gastrique acide mais entérosoluble, ladite substance étant choisie parmi l'acétophtalate de cellulose, l'acétophtalate de polyvinyle, les phtalates d'hydroxypropylméthylcellulose, les poly (éther méthylvinylique / anhydride maléïque), les acides choliques et les acides gras en $C_{10}$ à $C_{20}$ ; de sorte que la forme galénique est stable à la chaleur.

2. Forme galénique du vérapamil suivant la revendication 1, caractérisée en ce que le ou les polymères de type acrylique et/ou méthacrylique insolubles, constituant une partie du mélange filmogène de la membrane microporeuse, sont choisis parmi les copolymères ester éthylique d'acide acrylique/ester méthylique d'acide méthacrylique.

3. Forme galénique du vérapamil suivant la revendication 1 ou 2, caractérisée en ce que l'adjuvant contenu dans la membrane microporeuse est choisi parmi les suivants :
   - plastifiants, tels que la triacétine, le dibutylphtalate, le dibutylsébaçate, les esters d'acide citrique, les polyéthylèneglycols, les polypropylèneglycols et la polyvinylpyrrolidone ;
   - pigments éventuellement colorés, tels que les oxydes de fer et l'oxyde de titane ;
   - charges, telles que le lactose et le saccharose ;
   - mouillants, tels que des esters partiels d'acides gras (acides laurique, palmitique, stéarique et oléique) et d'anhydrides d'hexitols dérivés de sorbitol contenant éventuellement des chaînes polyoxyéthyléniques, de préférence les esters polyoxyéthylénés d'acides gras de sorbitane, ainsi que les polyéthylèneglycols ;
   - lubrifiants, tels que le stéréate de magnésium et le talc ;
   - agents anti-mousse, tels que l'huile de silicone ;
   - agents antistatiques, tels que l'oxyde d'aluminium colloïdal.

4. Forme galénique du vérapamil suivant l'une quelconque des revendications 1 à 3, caractérisée en ce que les microgranules contenant la substance active se présentent sous forme de sphérules, dont le diamètre est compris entre 0,05 mm et 3 mm, de préférence entre 0,1 mm et 2 mm.

5. Forme galénique du vérapamil suivant l'une quelconque des revendications précédentes, caractérisée en ce que les microgranules contiennent au moins un agent mouillant choisi parmi les suivants :
   - le saccharose, le mannitol, le sorbitol ;
   - les lécithines ;
   - les polyvinylpyrrolidones ;
   - les esters d'acide gras en $C_{12}$ à $C_{20}$ du saccharose ou sucroesters ;
   - les glycérides en $C_{12}$ à $C_{20}$ du saccharose ou sucroglycérides ;
   - les esters de xylose ou xylites ;
   - les glycérides polyoxyéthyléniques ;
   - les esters d'acides gras et de polyoxyéthylène ;
   - les éthers d'alcool gras et de polyoxyéthylène ;
   - les esters d'acides gras de sorbitane ;
   - les esters polyoxyéthylénés d'acides gras de sorbitane, et
   - les glycérides-polyglycides et esters d'alcoolspolyglycides.

6. Forme galénique du vérapamil suivant la revendication 1 ou 2, caractérisée en ce que la membrane microporeuse enrobant les microgranules contient, en poids, 5 à 70 % du mélange filmogène, 1 à 15 % de talc, 1 à 15 % de polyvinylpyrrolidone, 0,05 à 2 % d'oxyde d'aluminium colloïdal, 0,01 à 2 % d'esters polyoxyéthylénés d'acides gras de sorbitane et 0,01 à 2% d'huile de silicone.

7. Forme galénique du vérapamil suivant l'une quelconque des revendications précédentes, caractérisée en ce que le poids de la membrane microporeuse est de 2 à 35 %, de préférence de 5 à 22 %, de celui des microgranules qu'elle enrobe.

8. Médicament contenant du vérapamil à administrer par la voie buccale, caractérisé en ce qu'il contient une forme galénique de vérapamil suivant l'une quelconque des revendications précédentes, dans une gélule, un sachet, un distributeur de doses ou un comprimé.

9. Médicament suivant la revendication 8, caractérisé en ce qu'il se présente sous forme de doses unitaires contenant de 40 à 400 mg, de préférence de 80 à 360 mg de vérapamil sous forme de chlorhydrate, ou une quantité équivalente d'un autre sel.

10. Procédé de fabrication d'une nouvelle forme galénique du vérapamil suivant l'une quelconque des revendications 1 à 7, caractérisé en ce qu'il consiste à préparer au prélable des microgranules et à les enrober d'une membrane microporeuse, les microgranules et les membranes microporeuses présentant les compositions et caractéristiques indiquées dans l'une ou l'autre des revendications 1 à 7.

**Claims**

1. Galenic form of verapamil with prolonged release of this compound, consisting of microgranules containing a pharmacologically acceptable addition salt of verapamil with an acid, such as verapamil hydrochloride, as active substance, combined with at least one wetting agent, these microgranules being coated with a membrane, characterized in that the microgranules contain, apart from verapamil salt, at least one wetting agent selected from sugars, polyvinylpyrrolidone, $C_{12}$ to $C_{20}$ fatty acid esters of sucrose or of xylose, glycerides of sucrose, fatty acid esters of sucrose or of xylose, glycerides of sucrose, fatty acid ester of polyoxyethylene, ethers of fatty alcohols and polyoxyethylene, esters of sorbitan, esters of polyoxyethylene sorbitan, glyceride-polyglycides, alcohol-polyglycide esters and lecithins, and in that the microgranules are coated with a microporous membrane consisting of at least one pharmacologically acceptable adjuvant selected from plasticisers, pigments, fillers, wetting agents, lubricants, antistatic agents and antifoamants and at least one film forming mixture composed, on the one hand, of at least one polymer of the acrylic and/or methacrylic type which is insoluble in the organism and, on the other hand, of a substance which may be polymeric or not but is neither acrylic nor methacrylic and is insoluble in the acid gastric medium but soluble in the intestine, selected from cellulose acetophthalate, polyvinyl acetophthalate, hydroxypropylmethylcellulose phthalates, poly (methylvinyl ether/maleic anhydride) products, cholic acids and $C_{10}$ to $C_{20}$ fatty acids, so that the galenic form is thermically stable.

2. Galenic form of verapamil according to claim 1, characterized in that the insoluble polymer or polymers of the acrylic and/or methacrylic type constituting part of the film forming mixture of the microporous membrane are selected from acrylic acid ethylester/methacrylic acid methylester copolymers.

3. Galenic form of verapamil according to any one of the preceding claims, characterized in that the adjuvant contained in the microporous membrane is selected from the following :
   - plasticizers such as triacetine, dibutyl phthalate, dibutyl sebacate, citric acid esters, polyethylene glycols, polypropyleneglycols and polyvinylpyrrolidone ;
   - pigments, optionally coloured, such as oxides of iron and titanium dioxide ;
   - fillers such as lactose and sucrose ;
   - wetting agents such as partial esters of fatty acids (lauric, palmitic, stearic and oleic acid) and of hexitol anhydrides derived from sorbitol optionally containing polyoxyethylene chains, preferably polyoxyethylene esters of fatty acids of sorbitan, and polyethylene glycols ;
   - lubricants such as magnesium stearate and talc ;
   - antifoamants such as silicone oil ;
   - antistatic agents such as colloidal aluminium oxide.

4. Galenic form of verapamil according to any one of claims 1 to 3, characterized in that the microgranules containing the active substance are in the form of spheres having a diameter of from 0.05 to 3 mm, preferably from 0.1 mm. to 2 mm.

5. Galenic form of verapamil according to any one of the preceding claims, characterized in that the microgranules contain at least one wetting agent selected from the following :
   - sucrose, mannitol, sorbitol ;
   - lecithins ;
   - polyvinylpyrrolidones ;
   - $C_{12}$ to $C_{20}$ fatty acid esters of sucrose or sucroesters and crodestas ;
   - $C_{12}$ to $C_{20}$ glycerides of sucrose or sucroglycerides ;
   - esters of xylose or xylites ;
   - polyoxyethylene glycerides ;
   - esters of fatty acids and polyoxyethylene ;
   - ethers of fatty alcohols and polyoxyethylene ;
   - fatty acid esters of sorbitan ;
   - polyoxyethylene esters of fatty acids of sorbitan ; and
   - glyceride-polyglycides and esters of alcohol polyglycides.

6. Galenic form of verapamil according to claim 1 or 2, characterized in that the microporous membrane coating the microgranules contains, by weight, approximately 5 to 70% of the film forming mixture, 1 to 15% of talc, 1 to 15% of polyvinylpyrrolidone, 0.05 to 2% of colloidal aluminium oxide, 0.01 to 2% of polyoxyethylene esters of fatty acids of sorbitan and 0.01 to 2% of silicone oil.

7. New galenic form of verapamil according to any one of the preceding claims, characterized in that the weight of the microporous membrane is 2 to 35%, preferably 5 to 22% of that of the microgranules coated by the membrane.

8. Medicament containing verapamil for buccal administration, characterized in that it contains a galenic form of verapamil according to any one of the preceding claims in a capsule, a sachet, a dose distributor or a tablet.

9. Medicament according to claim 8, characterized in that it is prepared in the form of unit doses containing 40 to 400 mg, preferably 80 to 360 mg of verapamil in the form of its hydrochloride, or an equivalent quantity of another salt.

10. Process of manufacture of a new galenic form of verapamil according to any one of claims 1 to 7, characterized in that it consists of first preparing microgranules and then coating them with a microporous membrane, the microgranules and the microporous membranes having the compositions and characteristics indicated in one or other of claims 1 to 7.

**Patentansprüche**

1. Galenische Form von Verapamil mit verzögerter Freisetzung dieser Verbindung, bestehend aus Mikrokügelchen, die ein pharmakologisch annehmbares Säureadditionssalz von Verapamil, wie Verapamil-Chlorhydrat, als aktive Substanz, kombiniert mit mindestens einem Netzmittel, enthalten, wobei die Mikrokügelchen von einer Membran umhüllt sind, dadurch gekennzeichnet, daß
die Mikrokügelchen außer dem Verapamil-Salz mindestens ein Netzmittel, ausgewählt aus den Zukkern, Polyvinylpyrrolidon, den $C_{12}$- bis $C_{20}$-Fettsäureestern von Saccharose oder Xylose, den Saccharoseglyceriden, den Fettsäureestern von Polyoxyethylen, den Ethern von Fettalkoholen und Polyoxyethylen, den Sorbitanestern, den polyoxyethylenierten Sorbitanestern, den Polyglycid-glyceriden, den Estern von Alkoholen-polyglyciden und den Lecithinen, enthalten und daß
die Mikrokügelchen von einer mikroporösen Membran umhüllt sind, die aus mindestens einem pharmakologisch anhehmbaren Adjuvans, ausgewählt aus den Weichmachern, Pigmenten, Füllstoffen, Netzmitteln, Gleitmitteln, antistatischen Mitteln und Schaumbrechern, besteht sowie aus mindestens einem filmbildenden Gemisch, das zusammengesetzt ist aus einerseits mindestens einem im Organismus unlöslichen Polymeren vom Acryltyp und/oder Methacryltyp und andererseits aus einer Substanz, die polymer oder nicht polymer ist, aber nicht von acrylischer oder methacrylischer Beschaffenheit, die

im sauren Magensaft unlöslich, aber enterolöslich ist, und die aus Celluloseacetophthalat, Polyvinylacetophthalat, Hydroxypropylmethylcellulose-phthalaten, Poly(methylvinylether/maleinsäureanhydrid), Cholsäuren und $C_{10}$- bis $C_{20}$-Fettsäuren ausgewählt ist, so daß die galenische Form hitzebeständig ist.

**2.** Galenische Form von Verapamil nach Anspruch 1, dadurch gekennzeichnet, daß das Polymer oder die Polymeren vom Acryltyp und/oder Methacryltyp, die unlöslich sind und einen Teil des filmbildenden Gemisches der mikroporösen Membran ausmachen, unter den Acrylsäureethylester/Methacrylsäuremethylester-Copolymeren ausgewählt sind.

**3.** Galenische Form von Verapamil nach Anspruch 1 oder 2, dadurch gekennzeichnet, daß das in der mikroporösen Membran enthaltene Adjuvans unter folgenden Stoffen ausgewählt ist:
- Weichmacher, wie Triacetin, Dibutylphthalat, Dibutylsebacat, Citronensäureester, Polyethylenglykole, Polypropylenglykole und Polyvinylpyrrolidon;
- gegebenenfalls gefärbte Pigmente, wie Eisenoxide und Titanoxid;
- Füllstoffe, wie Lactose und Saccharose;
- Netzmittel, wie die partiellen Ester von Fettsäuren (Laurinsäure, Palmitinsäure, Stearinsäure und Ölsäure) mit Hexitolanhydriden, abgeleitet von Sorbit, die gegebenenfalls Polyoxyethylenketten enthalten, vorzugsweise polyoxyethylenierte Ester von Sorbitan-Fettsäuren, sowie Polyethylenglykole;
- Gleitmittel, wie Magnesiumstearat und Talk;
- Schaumbrecher, wie Siliconöl;
- antistatische Mittel, wie kolloidales Aluminiumoxid.

**4.** Galenische Form von Verapamil nach einem der Ansprüche 1 bis 3, dadurch gekennzeichnet, daß die Mikrokügelchen, die die aktive Substanz enthalten, als Kügelchen mit einem Durchmesser im Bereich von 0,05 bis 3 mm, vorzugsweise im Bereich von 0,1 mm bis 2 mm, vorliegen.

**5.** Galenische Form von Verapamil nach einem der vorangehenden Ansprüche, dadurch gekennzeichnet, daß die Mikrokügelchen mindestens ein Netzmittel, ausgewählt aus folgenden Stoffen, enthalten:
- Saccharose, Mannit, Sorbit;
- Lecithine;
- Polyvinylpyrrolidone;
- $C_{12}$- bis $C_{20}$-Fettsäureester von Saccharose oder Sucroester;
- $C_{12}$- bis $C_{20}$-Saccharoseglyceride oder Sucroglyceride;
- Xyloseester oder Xylite;
- Polyoxyethylenglyceride;
- Polyoxyethylen-Fettsäureester;
- Ether aus Fettalkoholen und Polyoxyethylen;
- Sorbitan-Fettsäureester;
- polyoxyethylenierte Sorbitan-Fettsäureester und
- Polyglycid-glyceride und Polyglycid-alkoholester.

**6.** Galenische Form von Verapamil nach Anspruch 1 oder 2, dadurch gekennzeichnet, daß die mikroporöse Membran, die die Mikrokügelchen umhüllt, bezogen auf das Gewicht, 5 bis 70% filmbildendes Gemisch, 1 bis 15% Talk, 1 bis 15% Polyvinylpyrrolidon, 0,05 bis 2% kolloidales Aluminiumoxid, 0,01 bis 2% polyoxyethyleniertes Sorbitan-Fettsäureester und 0,01 bis 2% Siliconöl enthält.

**7.** Galenische Form von Verapamil nach einem der vorangehenden Ansprüche, dadurch gekennzeichnet, daß das Gewicht der mikroporösen Membran 2 bis 35%, vorzugsweise 5 bis 22%, des Gewichtes der Mikrokügelchen, die sie umhüllt, ausmacht.

**8.** Arzneimittel, enthaltend Verapamil, zur oralen Verabreichung, dadurch gekennzeichnet, daß es eine galenische Form von Verapamil nach einem der vorangehenden Ansprüche in einer Gelkapsel, einem Briefchen, einem Dosisspender oder eienr Tablette enthält.

**9.** Arzneimittel nach Anspruch 8, dadurch gekennzeichnet, daß es in Dosiseinheiten vorliegt, die 40 bis 400 mg, vorzugsweise 80 bis 360 mg, Verapamil in Form des Chlorhydrats oder eine äquivalente Menge eines anderen Salzes enthalten.

10. Verfahren zur Herstellung einer neuen galenischen Form von Verapamil nach einem der Ansprüche 1 bis 7, dadurch gekennzeichnet, daß man zunächst Mikrokügelchen herstellt und sie dann mit einer mikroporösen Membran umhüllt, wobei die Mikrokügelchen und die mikroporösen Membranen die in einem der Ansprüche 1 bis 7 angegebenen Zusammensetzungen und Merkmale aufweisen.